# EUROPEAN PATENT SPECIFICATION

(11) **EP 1 728 528 B1**
(45) Date of publication and mention of the grant of the patent: **19.06.2013**
(21) Application number: 05721165.8
(22) Date of filing: 18.03.2005
(51) Int. Cl.: A61M 5/24

(54) **PRE-FILLED SYRINGE**
FERTIGSPRITZEN
SERINGUE PRE-REMPLIE

(30) Priority: 23.03.2004 JP 2004083875
(43) Date of publication of application: 06.12.2006
(73) Proprietor: NIPRO CORPORATION, Osaka-shi, Osaka 531-8510 (JP)
(72) Inventor: Hasegawa, Mitsuru, Osaka-shi, Osaka 5318510 (JP)
(74) Representative: Banzer, Hans-Jörg
(86) International application number: PCT/JP2005/005015
(87) International publication number: WO 2005/089837

(56) References cited:
- EP-A- 0 172 990
- EP-A- 0 737 485
- EP-A- 0 803 261
- WO-A-03/015854
- WO-A1-95/17916
- WO-A1-97/05916
- JP-A- 11 332 984
- JP-A- 11 332 984
- JP-A- 61 048 377

## Description

### Technical Field

The present invention relates to a pre-filled syringe. More particularly, it relates to a two-component mixing type pre-filled syringe in which an inside of a barrel formed with a bypass is partitioned into two chambers, forward and rearward, by a gasket, and whose total length is comparatively short.

### Background Art

When a medicine of which stability is difficult to maintain from the viewpoints of preparation and medicinal effect is mixed or dissolved, the medicine has been hitherto previously and separately stored in an ampule or vial and the like until just before use, and prepared and used with a syringe or an injection needle and the like at the time of use. However, this method requires a lot of time and effort for the preparation because it is troublesome in its operation and, in addition, there is fear of contamination with bacteria floating in the air, significant fear that a foreign matter such as a piece of glass in the ampule and a piece of rubber in the vial is mixed with the medicine. Whereupon, in recent years, as one means of solving such problem, a so-called two-component mixing type pre-filled syringe, in which two different medicines are stored in one syringe while being mutually separated and mixed at the time of use, is put to practical use.

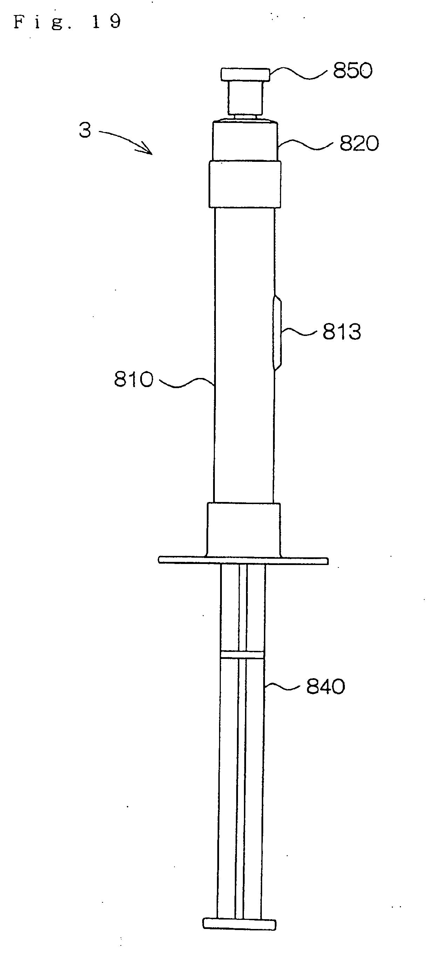

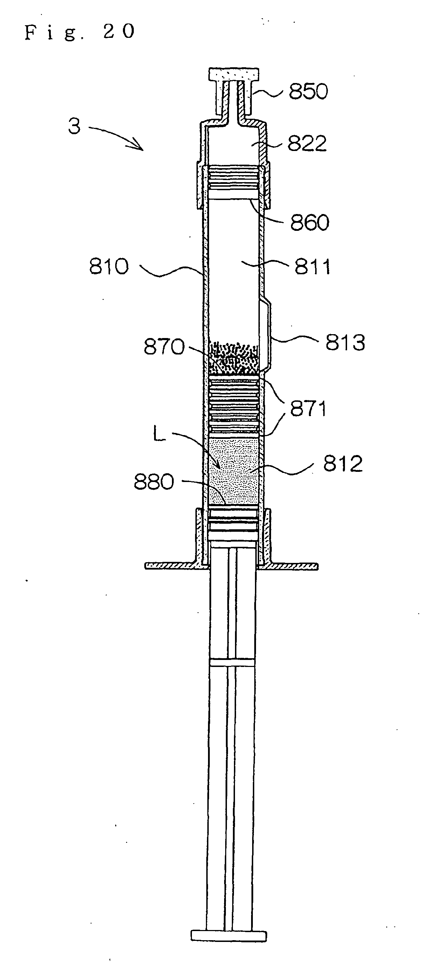

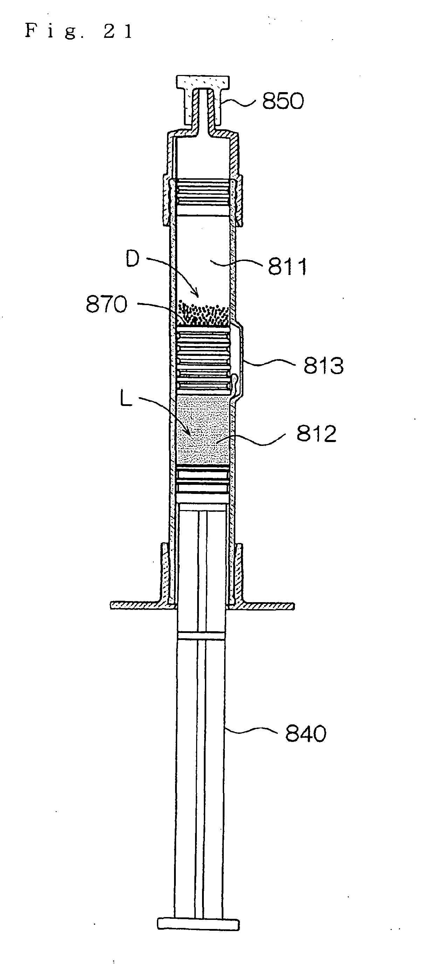

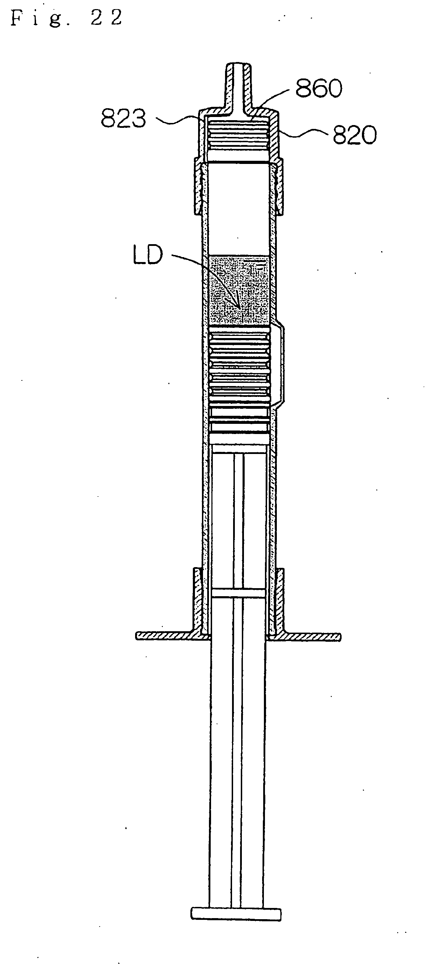

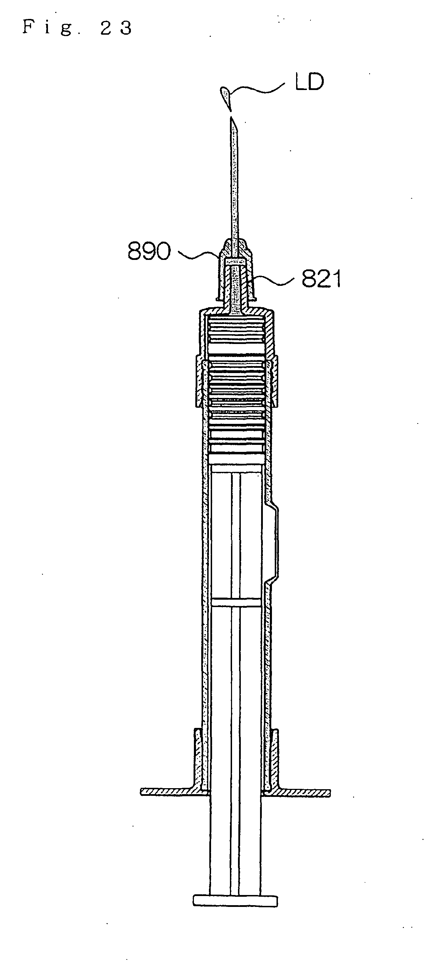

The two-component mixing type pre-filled syringe marketed at present is one as shown, for example, in Patent Document 1. This conventional two-component mixing type pre-filled syringe is explained in detail by referring to Fig. 19 to Fig. 23. Fig. 19 is a front view, and Fig. 20 is a sectional view. Fig. 21 to Fig. 23 are views illustrating use of the syringe. That is, as shown in Fig. 20, a conventional two-component mixing type pre-filled syringe 3 comprises a structure in which an inside of a barrel 810 is separated into front-and-rear chambers 811 and 812 by a tip gasket 860, an intermediate gasket 870 and a plunger gasket 880, and a bypass 813 is concavely provided in an inner wall of the barrel 810. And, as shown in Fig. 21, when a plunger rod 840 is pushed in, the intermediate gasket 870 advances to a position of the bypass 813, and a dissolving liquid L in the rear chamber 812 enters into the front chamber 811 through the bypass 813 and is mixed with a powdery medicine D. Thereafter, a cap is removed and the plunger rod 840 is additionally pushed and advanced as shown in Fig. 22, and the tip gasket 860 is advanced to inside of nozzle member 820. Since a liquid passing passage 823 is concavely provided in an inner wall of the nozzle member 820, a mixed medicine LD is administered by connecting an injection needle 890 or the like to nozzle 821 as shown in Fig. 23.

Patent Document 1: JP-A-10-211280 Gazette (Fig. 1)

### Disclosure of the Invention

### Problems to be solved by the Invention

However, in the conventional two-component mixing type pre-filled syringe 3, since a seal part 871 is formed from a tip to a base end of the intermediate gasket 870 as shown in Fig. 20, in order that the liquid medicine L in the rear chamber 812 flow into the front chamber 811, an axial length of the bypass 813 must be longer than a total length of the intermediate gasket 870. For this reason, the length of the pre-filled syringe 3 becomes long.

Further, the intermediate gasket 870 must advance by at least its total length, since the rear chamber 812 cannot communicate with the bypass 813, and the distance of movement becomes long. If the distance of movement is long, a pressure in the front chamber 811 greatly increases and, depending on conditions, there has been a fact that the tip gasket 860 moves during a mixing operation into the tip gasket accommodation part 822, the cap 850 flies off and thus an inside of the front chamber 811 is exposed to the air and contaminated, or that the mixing of the medicine cannot be smoothly performed. In order to avoid this fear, a pressure increase is prevented by lengthening a total length of the front chamber 811. As a result, an increase in the length of the pre-filled syringe 3 is also brought about.

Further, if the total length of the pre-filled syringe is long, that is, since the length of the plunger also becomes long, there occurs also a problem that operation of the syringe during administration of medicine is difficult. This problem becomes significant, especially in a case of a nurse whose hand is small.

EP 0 737 485 A1 discloses a syringe with two chambers separated by a stopper. The syringe contains a communication passage and when the stopper is located at the communication passage the content of the two chambers can mix.

JP 11-332984 A discloses a syringe having a hollow groove by which the content can pass a sealing provided in the front part of the syringe.

### Means for Solving the Problems

Whereupon, the present inventor has thought of the present invention as a result of earnestly repeating studies in order to solve the above-mentioned problems especially to allow a good control of the mixing of the powder and the liquid. That is, the present invention relates to:
(1) A pre-filled syringe which comprises a barrel having a tip in which a nozzle is provided and an opened base end, an intermediate gasket liquid-tightly partitioning an inside of the barrel into a front chamber and a rear chamber, a plunger gasket located in a base end side relative to the intermediate gasket and sealing the inside of the barrel, and a plunger rod connected to a base end of the plunger gasket, and in which in a tip side of the barrel relative to the intermediate gasket there is formed a bypass protruding outwardly in a radial direction,
wherein the intermediate gasket includes a seal part contacting an inner wall of the barrel and liquid-tightly partitioning the front chamber and the rear chamber, and a bypass communication passage providing communication between the front chamber and the rear chamber in cooperation with the bypass;

When an axial length of the bypass is a1 and an axial effective length of the seal part is b1, a1 > b1;

The bypass communication passage includes a circumferential groove formed in a base end side of the seal part in an approximately circumferential direction, and a connection passage connecting the circumferential groove and the rear chamber.

According to the invention the connection passage is a spiral groove having been formed in an outer wall of the intermediate gasket;
(2) A pre-filled syringe according to (1), wherein if an axial length of a tip gasket is A, an axial length of the intermediate gasket is B, an axial length of the plunger gasket is C and a length from an inner wall tip of a nozzle member to an inner wall base end of the bypass is D, A + B + C < D.
(3) A pre-filled syringe according to any of (1) or (2), wherein the barrel additionally comprises a tip gasket, and the front chamber is formed between the tip gasket and the intermediate gasket;
(4) A pre-filled syringe according to (3), wherein the barrel additionally comprises a nozzle member, the nozzle is formed in a tip of the nozzle member, and the nozzle member includes a tip gasket accommodation part capable of accommodating the tip gasket, and a liquid passing passage through which a medicine liquid can pass when the tip gasket has been accommodated in the tip gasket accommodation part; and the like.

### Advantage of the Invention

According to the present invention, without causing deterioration of storage stability of the medicine in a conventional two-component mixing type pre-filled syringe, it is possible to, by comparatively shortening the total length of the pre-filled syringe, provide a two-component mixing type pre-filled syringe which requires less storage place, and additionally solves the problem that the operation at the time of medicine administration is difficult to perform, and whose operability is also improved.

### Brief Description of the Drawings

Fig. 1 is a front view of a pre-filled syringe of example 1.
Fig. 2 is a sectional view of the pre-filled syringe of example 1.
Fig. 3 is a view explaining use of the pre-filled syringe of example 1.
Fig. 4 is a view explaining use of the pre-filled syringe of example 1.
Fig. 5 is a view explaining use of the pre-filled syringe of example 1.
Fig. 6 is an enlarged sectional view of the pre-filled syringe of example 1.
Fig. 7 is a view (side view and expansion) showing an intermediate gasket of example 1.
Fig. 8 is a sectional view in which the pre-filled syringe of example 1 and a conventional article are compared.
Fig. 9 is a front view of a pre-filled syringe of example 2.
Fig. 10 is a sectional view of the pre-filled syringe of example 2 not forming part of the invention.
Fig. 11 is a sectional view explaining use of the pre-filled syringe of example 2.
Fig. 12 is a sectional view seen from arrow line A - A of the pre-filled syringe in Fig. 11.
Fig. 13 is a view (side view and expansion) showing an intermediate gasket of example 2.
Fig. 14 is a view (side view and expansion) showing an intermediate gasket of an example 3.
Fig. 15 is a view (side view and expansion) showing an intermediate gasket of an example 4 not forming part of the invention.
Fig. 16 is a view (side view and expansion) showing an intermediate gasket of an example 5 not forming part of the invention.
Fig. 17 is a view (side view and expansion) showing an intermediate gasket of an example 6.
Fig. 18 s a view (side view and expansion) showing an intermediate gasket of an example 7.
Fig. 19 is a front view of a conventional two-component mixing type pre-filled syringe.
Fig. 20 is a sectional view of the conventional two-component mixing type pre-filled syringe.
Fig. 21 is a sectional view showing a use situation of the conventional two-component mixing type pre-filled syringe.
Fig. 22 is a sectional view showing use of the conventional two-component mixing type pre-filled syringe.
Fig. 23 is a sectional view showing use of the conventional two-component mixing type pre-filled syringe.

### Best Mode for Carrying Out the Invention

Next, examples of the present invention are explained on the basis of the drawings.

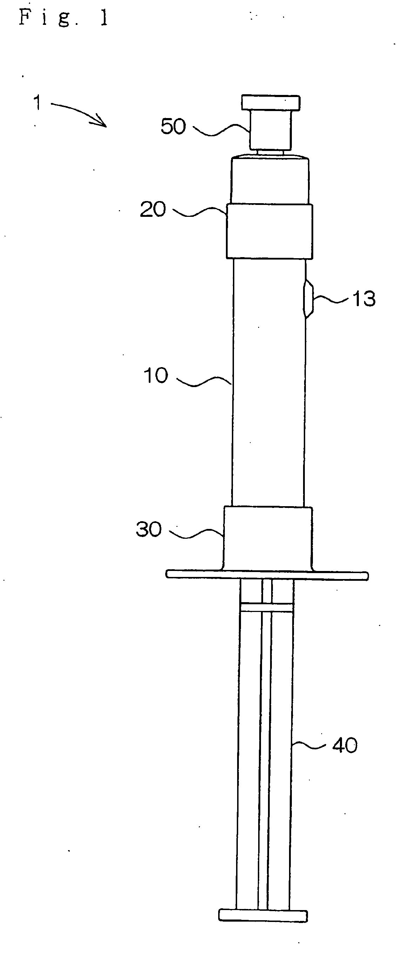

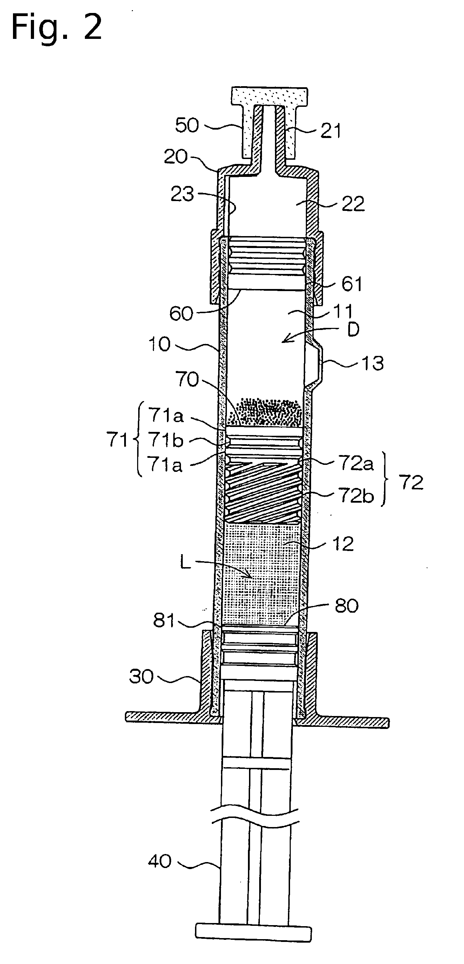

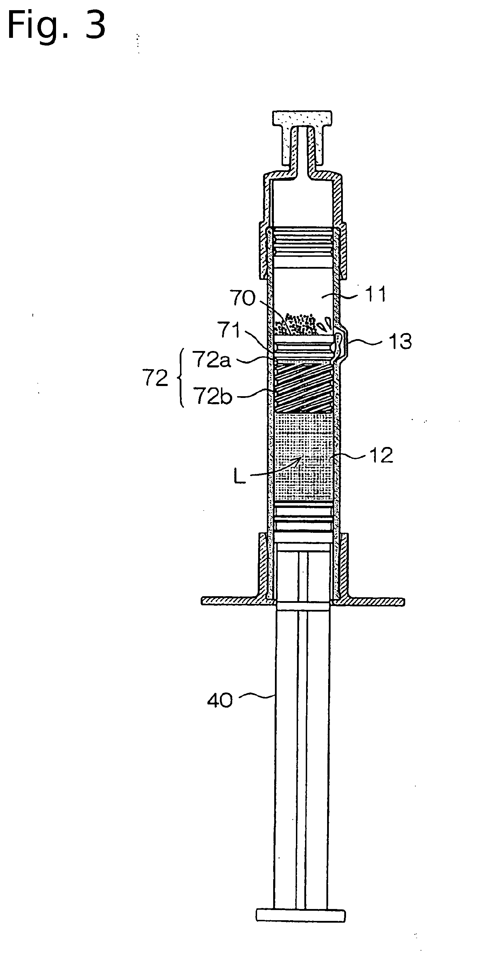

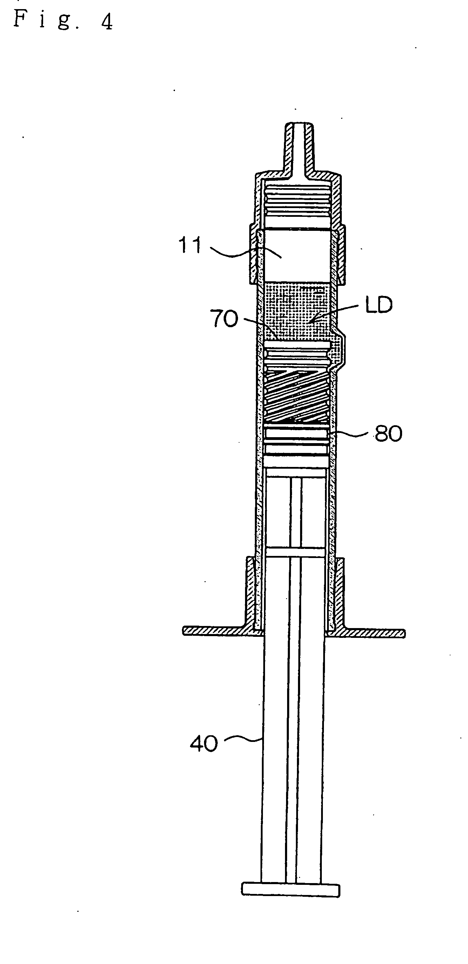

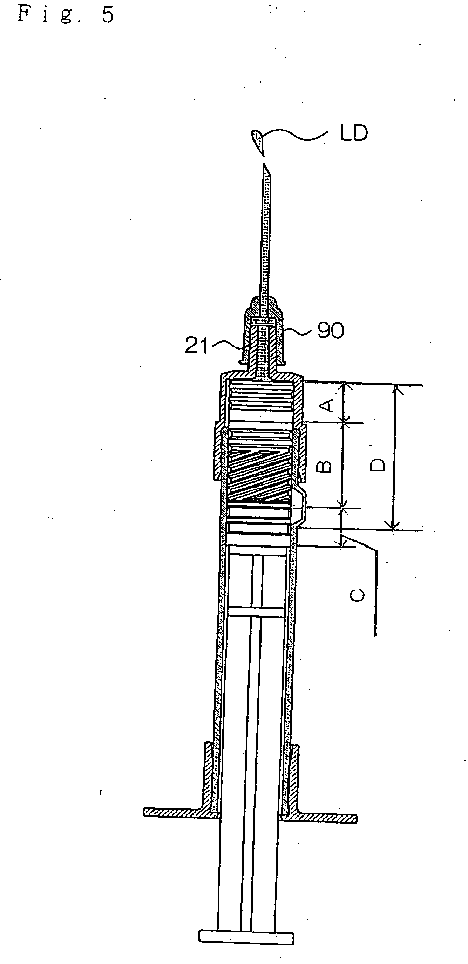

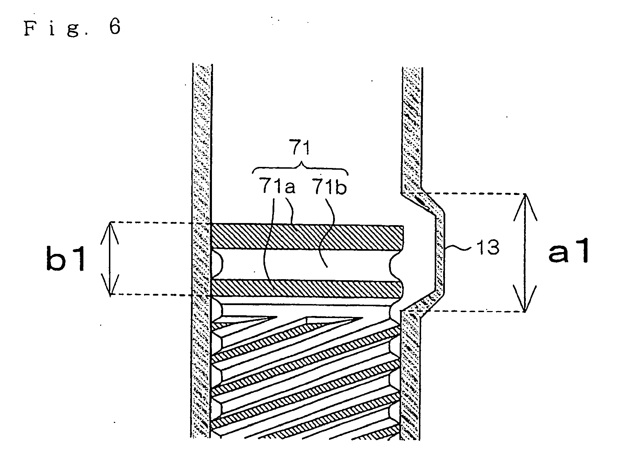

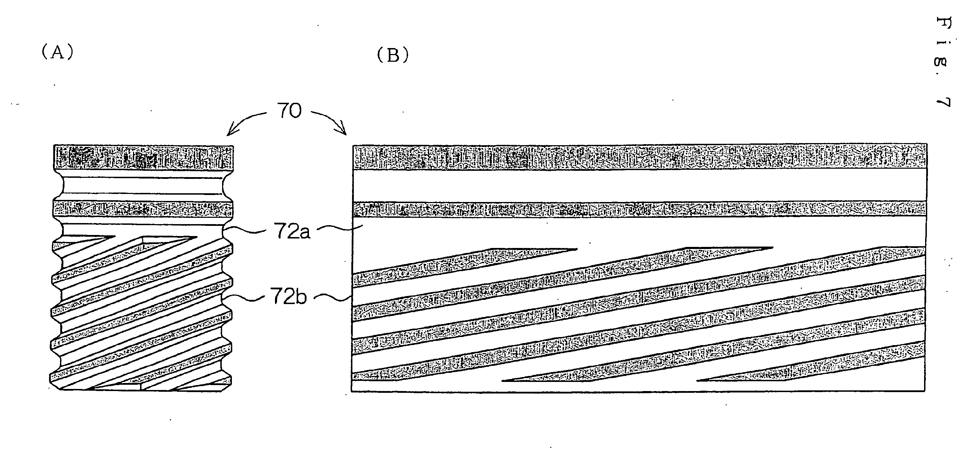

Fig. 1 to Fig. 7 are views showing a pre-filled syringe of an example 1 of the present invention. Fig. 1 is a front view of the same, Fig. 2 is a sectional view of the same and Fig. 3 to Fig. 5 are sectional views explaining a use situation of the pre-filled syringe of example 1. Fig. 3 is a view showing a state that an intermediate gasket advances to a bypass and a liquid medicine in a rear chamber flows into a front chamber, Fig. 4 is a view showing a state that mixing of medicines has been completed, and Fig. 5 is a view in which the mixed medicine is being discharged. Fig. 6 is an enlarged sectional view of the bypass communication passage of Fig. 3. Fig. 7 is a view showing an intermediate gasket of example 1. In Fig. 7, (A) is a side view, and (B) shows a view in which a side face has been expanded. The shaded portion of Fig. 7 shows a face contacting a barrel.

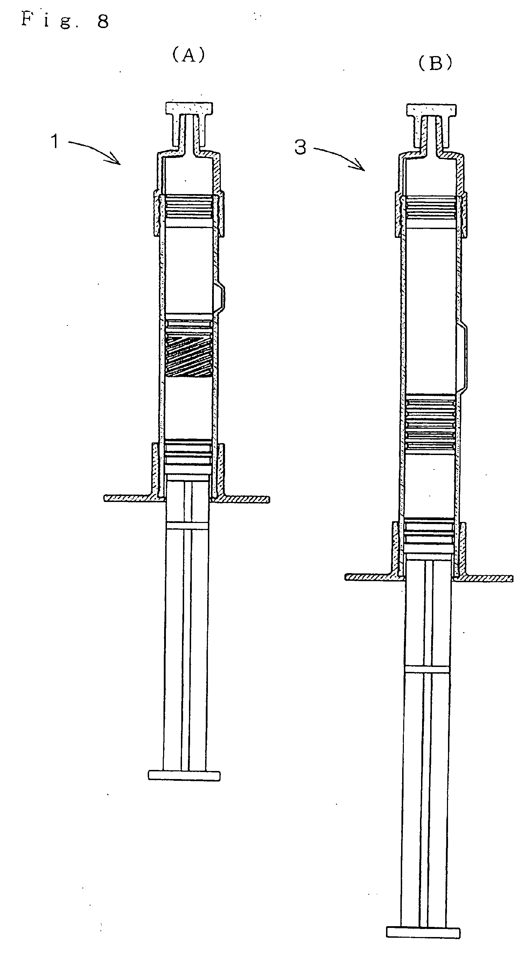

Fig. 8 is a comparison view of example 1 of the presentinvention and a conventional article. In Fig. 8, (A) is a two-component mixing type pre-filled syringe of the present invention, (B) is a conventional two-component mixing type pre-filled syringe, and they respectively show a state before being used.

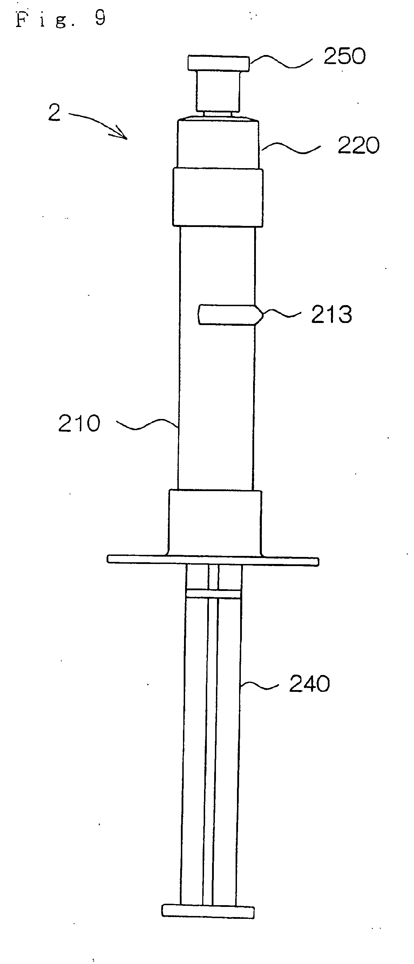

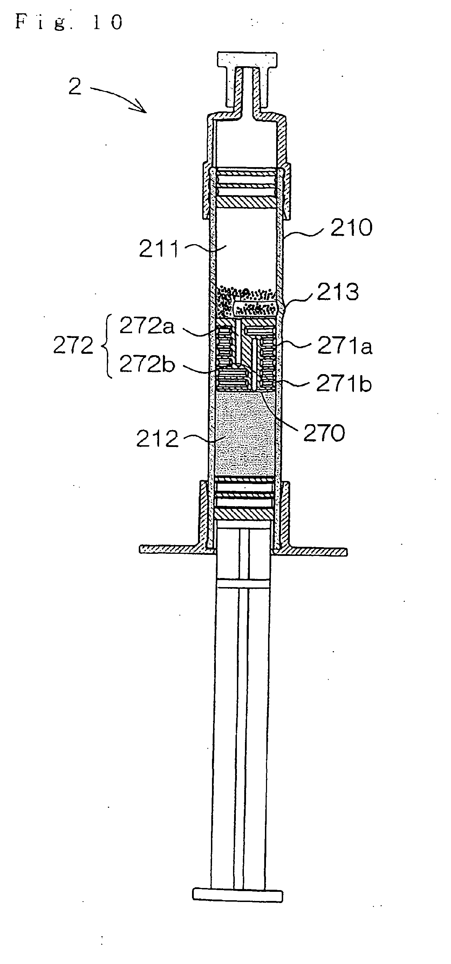

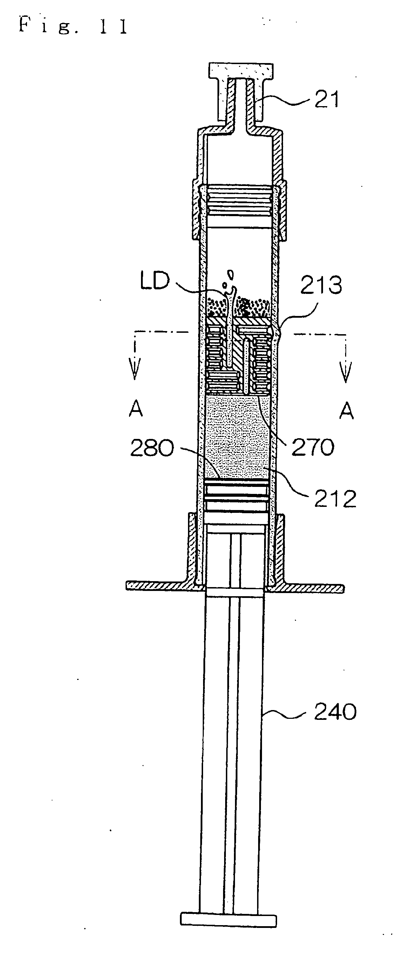

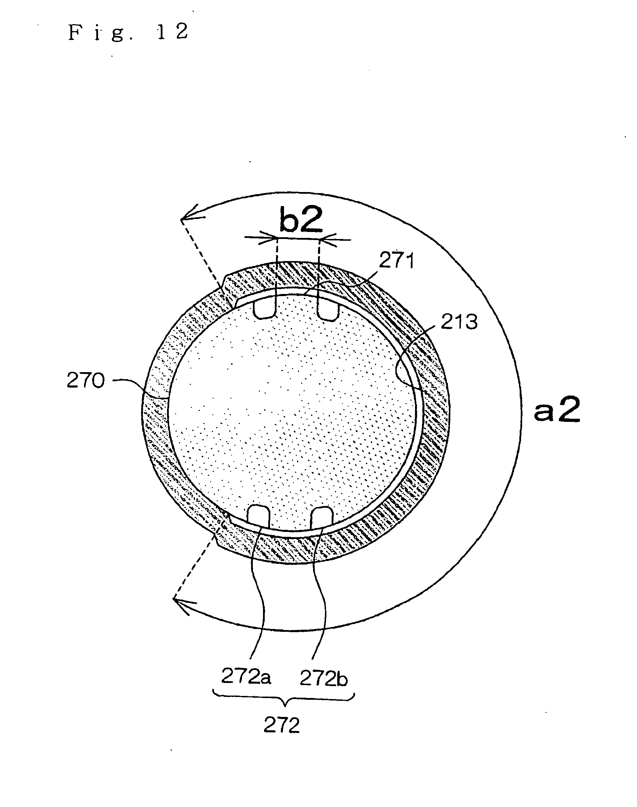

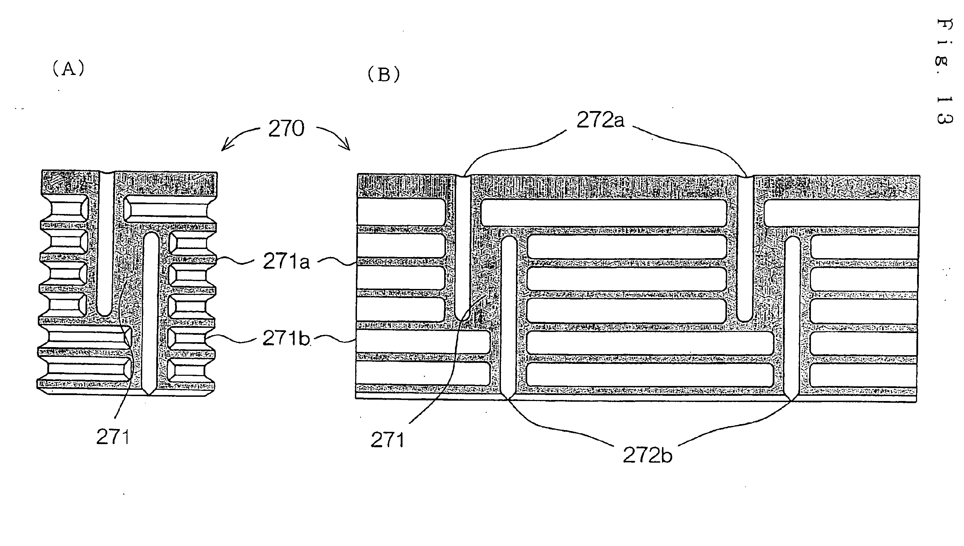

Fig. 9 to Fig. 13 are views showing a pre-filled syringe of example 2 of the present invention. Fig. 9 is a front view of the same, and Fig. 10 is a sectional view of the same. Fig. 11 is a sectional view explaining use of the pre-filled syringe of example 2, and a view showing a state that a liquid medicine in a rear chamber flows into a front chamber through a bypass communication passage and a communication passage which have been connected via a bypass. Fig. 12 is a view seen from arrow line A - A in Fig. 11. Fig. 13 is a view showing an intermediate gasket of example 2. In Fig. 13, (A) is a side view, and (B) shows a view in which a side face has been expanded. The shaded portion of Fig. 13 shows a face contacting a barrel.

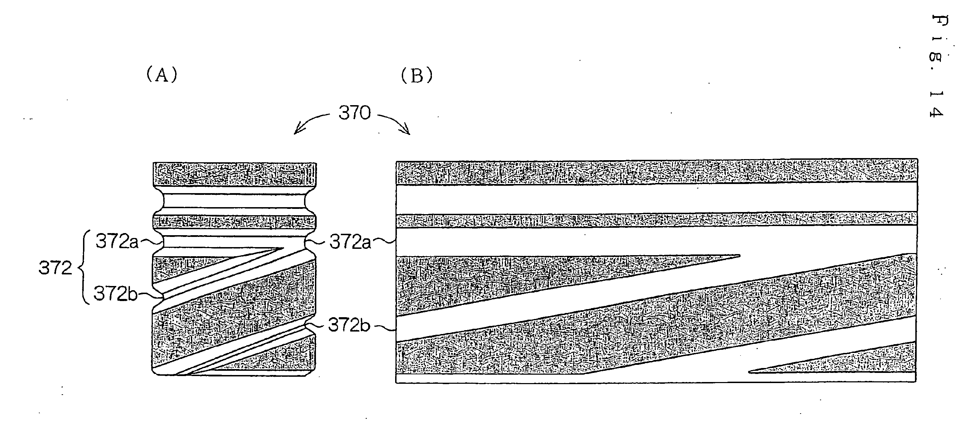

Fig. 14 to Fig. 18 are views showing intermediate gaskets of other examples and, in Figs. 14, 15, 17 and 18, (A) shows a side view and (B) a side face expansion.

Fig. 14 is a view showing an intermediate gasket of example 3, and a connection passage becomes one thread groove. The shaded portion in the drawing shows a face contacting with a barrel.

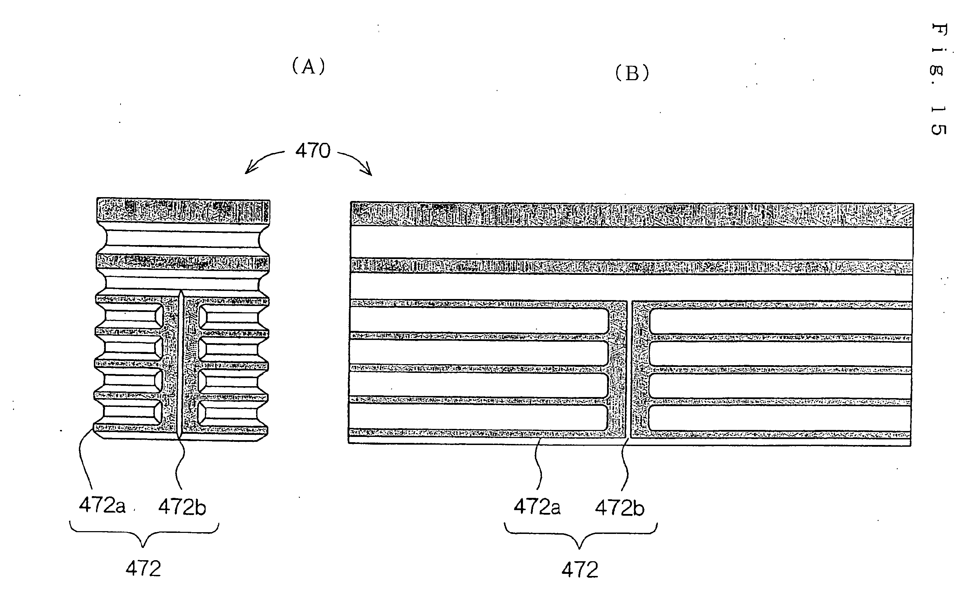

Fig. 15 is a view showing an intermediate gasket of example 4, and a connection passage becomes a linear groove parallel to an axial direction. The shaded portion in the drawing shows a face contacting with a barrel.

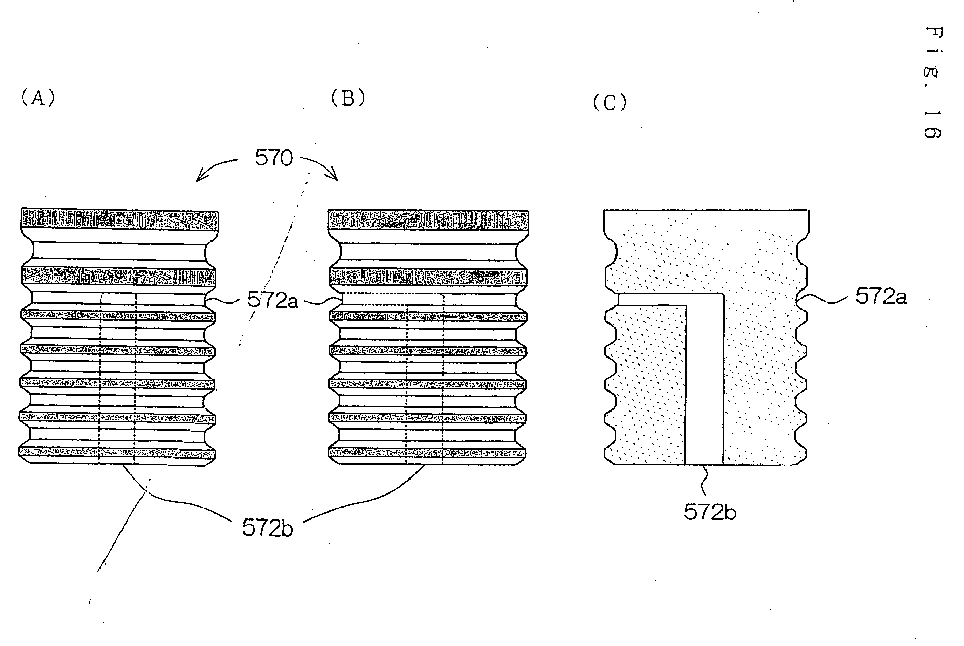

Fig. 16 is a view showing an intermediate gasket of example 5, and a connection passage becomes an approximately L-shaped conduit formed inside the gasket. (A) shows a front view, (B) a right side view, and (C) a sectional view of (B). The shaded portion in the drawing shows a face contacting a barrel.

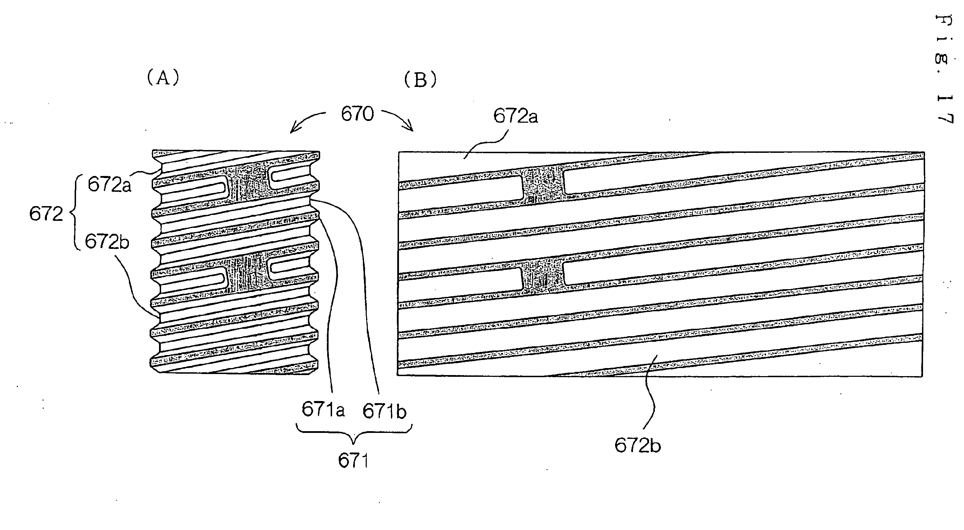

Fig. 17 is a view showing an intermediate gasket of example 6, and there is formed a bypass communication passage comprising spiral first and second grooves respectively going in reverse directions from a tip and a base end. The shaded portion in the drawing shows a face contacting a barrel.

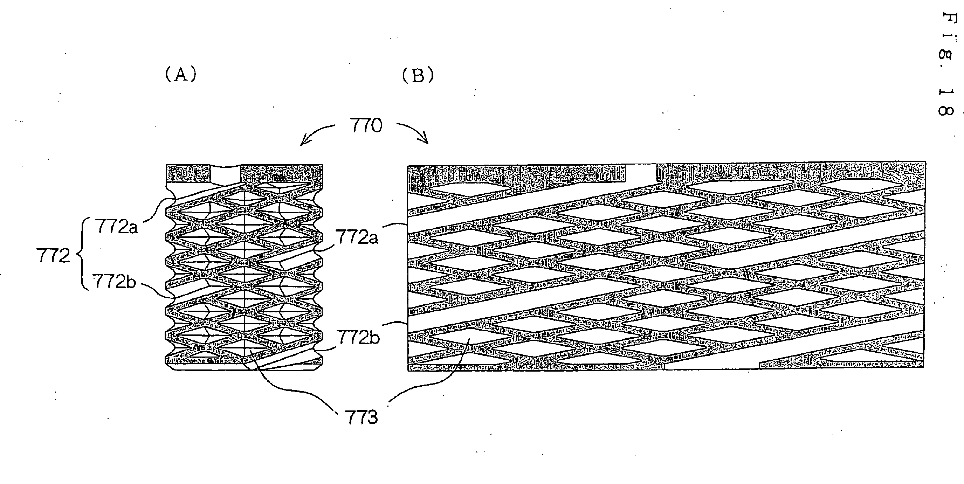

Fig. 18 is a view showing an intermediate gasket of example 7, and there is formed a bypass communication passage comprising spiral first and second grooves respectively going in reverse directions from a tip and a base end, and a depression is formed in a seal part except for the bypass communication passage of an intermediate gasket side wall. The shaded portion in the drawing shows a face contacting a barrel.

### Example 1

It is desirable that a pre-filled syringe 1 that is one implementation mode of the present invention has, among the above-mentioned characteristics, (1), (2), (3), (4) and (5), and additionally (9), (10) and (11). Concretely, as shown in Fig. 1 to Fig. 7, the pre-filled syringe 1 comprises a nozzle member 20 provided with a nozzle 21 sealed by a cap 50, a barrel 10 whose tip end has been liquid-tightly inserted and fixed into a base end of the nozzle member 20 and whose base end has been opened, a tip gasket 60 sealing a tip side of the barrel 10, an intermediate gasket 70 liquid-tightly partitioning an inside of the barrel 10 into a front chamber 11 and a rear chamber 12, a plunger gasket 80 which is located in a base end side relative to the intermediate gasket 70 and seals the inside of the barrel 10, and a plunger rod 40 connected to a base end of the plunger gasket 80, and comprises a construction in which, in a tip side relative to the intermediate gasket 70 of the barrel 10, there is formed a bypass 13 protruding outwardly in a radial direction , the liquid medicine L (e.g., dissolving liquid) is accommodated in the rear chamber 12, the intermediate gasket 70 contacts an inner wall of the barrel 10, and there are included at least one seal part 71 which liquid-tightly partitions the front chamber 11 and the rear chamber 12, and a bypass communication passage 72 which provides communication between the front chamber 11 and the rear chamber in cooperation with the bypass 13.

The nozzle member 20 is a tubular member which has in its tip the nozzle 21 capable of discharging the medicine liquid LD and whose base end is opened, and into the base end of which it is possible to liquid-tightly insert and fix the barrel 10. There is formed in its inside a later-mentioned tip gasket accommodation part 22 capable of accommodating the tip gasket 60 . Further, as shown in Fig. 4 and Fig. 5, in an inner wall of the tip gasket accommodation part 22 there is formed a liquid passing passage 23 through which the medicine liquid LD can pass when the tip gasket 60 has been accommodated in the tip gasket accommodation part 22. This liquid passing passage 23 is a groove having been concavely provided in a side wall and a top face of the tip gasket accommodation part 22, and its one end is connected to a hollow inside of the nozzle 21 and the other end can communicate with barrel 10. Incidentally, an axial length of the liquid passing passage 23 must be longer than an axial length of the tip gasket 60 and, if it is short, it is impossible to discharge the medicine liquid LD. Further, as shown in Fig. 5, the nozzle 21 can be formed into a male luer to which an injection nozzle 90 can be connected.

Further, as a material for forming the nozzle member 20, although there are exemplified, for example, polyolefin resins such as polyethylene and polypropylene, polyvinyl chloride, PET (polyethylene terephthalate), EVA (ethylene-vinyl acetate copolymer), EVOH (ethylene-vinyl alcohol copolymer), polyamide, polyvinylidene chloride, polyvinyl fluoride, polytrifluorchloroethylene, polyester, nylon, mixtures thereof and laminated bodies thereof, the raw material is not especially limited if it is useful as a medical equipment material, does not interact with the medicine accommodated in the barrel 10, and there is no fear of elution into the medicine or the like.

The cap 50 is closely attached so as to seal the nozzle 21 and, as a material for forming it, although there can be suitably adopted an elastic material such as, for example, butyl rubber, silicone rubber, thermoplastic elastomer and silicone elastomer, the raw material is not especially limited if it is useful as a medical equipment material and can seal the nozzle.

The barrel 10 is a tubular member whose tip and base end are opened, a finger-applying flange 30 is attached to the base end, and the tip is liquid-tightly inserted and fixed into the nozzle member 20. The tip of the barrel 10 is liquid-tightly sealed inside of the barrel 10 by the tip gasket 60, and the base end is liquid-tightly sealed by the plunger gasket 80. The intermediate gasket 70 is inserted in the barrel 10, and the inside of the barrel 10 is liquid-tightly partitioned into the front chamber 11 and the rear chamber 12 by the intermediate gasket 70. Additionally, in a wall of the front chamber 11 there is formed the bypass 13 in an axial direction of the barrel 10 so as to protrude outwardly in the radial direction. As a material for forming the barrel 10, although there are exemplified, for example, glass, polyolefin resin such as polyethylene and polypropylene, polyvinyl chloride, PET (polyethylene terephthalate), EVA (ethylene-vinyl acetate copolymer), EVOH (ethylene-vinyl alcohol copolymer), polyamide, polyvinylidene chloride, polyvinyl fluoride, polytrifluorchloroethylene, polyester, nylon, mixtures thereof and a laminated body thereof, the raw material is not especially limited if it is useful as a medical equipment material, does not interact with the medicine accommodated in the barrel 10, and there is no fear of elution into the medicine or the like.

In the rear chamber 12 there is accommodated the liquid medicine L. As the liquid medicine accommodated in the rear chamber 12, such liquid as physiological salt solution and glucose solution or a medicinal liquid is desirable. The powdery medicine D is accommodated in the front chamber 11. The medicine accommodated in the front chamber 11 is a powdery medicine, for example, a lyophilized preparation such as an antibiotic, which is unstable if it is dissolved in the liquid medicine L in the rear chamber 12.

Each of the tip gasket 60, the plunger gasket 80 and the intermediate gasket 70 is a tubular body consisting of an elastic material and, as a material for forming it, although there can be suitably adopted an elastic material such as, for example, butyl rubber, silicone rubber, thermoplastic elastomer and silicone elastomer, the raw material is not especially limited if it is one which is useful as the medical equipment material, and does not interact with the medicine accommodated in the barrel 10.

The tip gasket 60 and the plunger gasket 80 have several annular ribs 61 and 81 in their side faces, which are formed in a circumferential direction and liquid-tightly contact the inner wall of the barrel 10, and they can slide inside the barrel 10. Further, as a material for forming them, although there can be suitably adopted an elastic material such as, for example, butyl rubber, silicone rubber, thermoplastic elastomer and silicone elastomer, the raw material is not especially limited if it is useful as a medical equipment material, and can liquid-tightly seal the inside of the barrel 10.

The plunger rod 40 is connected to the base end of the plunger gasket 80. The plunger rod 40 may be previously connected, or may be connected when used. As a connecting method, there are exemplified a fitting, a meshing and the like. Further, as a material for forming the plunger rod 40, although there are exemplified a synthetic resin and the like, the raw material is not especially limited.

The intermediate gasket 70 of the present invention is characterized by comprising a construction which includes the bypass communication passage 72 consisting of seal part 71 liquid-tightly contacting the inner wall of the barrel 10 in its tip side, a circumferential groove 72a formed in a circumferential direction in a base end side of the seal part 71, and a connection passage 72b connecting the circumferential groove 72a and the rear chamber 12. Incidentally, the seal part 71 of example 1 is constituted by two annular ribs 71a. That is, the seal part mentioned in the present invention is a seal which substantially partitions the front chamber and the rear chamber, and may include a portion 71b which does not contact the inner wall of the barrel 10. The annular ribs 71a are not limited to two. An annular groove 671b of example 6 shown in Fig. 17 or a depression 773 of example 7 shown in Fig. 18 is also a portion which similarly does not contact the inner wall of the barrel.

As shown in (A) and (B) of Fig. 7, the connection passage 72b of the present invention is constituted by a spiral thread groove, and one end of the connection passage 72b is connected to the circumferential groove 72a and the other end is connected to a bottom face of the intermediate gasket 70. By making the connection passage 72b into the spiral thread groove, since it is possible to lengthen a distance between the circumferential groove 72a and the rear chamber 12, it is possible to more liquid-tightly partition the front chamber 11 and the rear chamber 12. Further, when a hydrophilic liquid medicine is accommodated in the rear chamber 12, since it is possible to prevent entry of the liquid medicine L into the bypass communication passage 72 by a hydrophobic coating in an inside of the bypass communication passage 72, it is possible to more liquid-tightly partition the front chamber 11 and the rear chamber 12. As a hydrophilic coating agent, although it is possible to suitably use a water-soluble or hydrophilic polymeric material such as, for example, polyethylene glycol, polyethylene oxide, polyvinyl alcohol, polyvinyl pyrrolidone, polyacrylamide, collagen, chitosan, methyl vinyl ether - maleic anhydride, and copolymers and derivatives thereof, it is of course not limited to these. Incidentally, in a case where the liquid medicine L is hydrophobic, it suffices if, conversely, a hydrophilic coating is applied to the inside of the bypass connection passage 72b.

As shown in Fig. 6, in the present invention, when an axial length of the bypass 13 is a1, and an axial effective length of the seal part 71 is b1, a1 > b1. In this manner, when the intermediate gasket 70 is moved forward as shown in Fig. 3, the liquid medicine L in the rear chamber 12 flows over the seal part 71 and can flow into the front chamber 11 through the bypass 13. The axial effective length of the seal part 71 means, between the two annular ribs 71a, a distance from a tip part of the tip side rib 71a to a base end part of the rear end side rib 71a.

In the present invention, by shortening the effective length of the seal part 71 of the intermediate gasket 70, and also shortening the length of the bypass 13, and providing the bypass communication passage 72 including the connection passage 72b, which is spirally and lengthily extended, in the base end side of the seal part 71, it is possible as shown in Fig. 8 to provide a two-component mixing type pre-filled syringe 1 which is shorter than a conventional two-component mixing type pre-filled syringe 3, and does not impair the stability of the medicine while improving operability.

As shown in Fig. 5, in the present invention, if an axial length of the tip gasket 60 is A, an axial length of the intermediate gasket 70 is B, an axial length of the plunger gasket 80 is C and a length from an inner wall tip of the nozzle member 20 to an inner wall base end of the bypass 13 is D, it is desirable that A + B + C > D. In this manner, the most base end side annular rib 81 of the plunger gasket 80 having several annular ribs 81 becomes so as to be always located in the base end side relative to the base end within the inner wall of the bypass 13.

Subsequently, there is explained about a method of using the pre-filled syringe 1 of example 1 by referring to Fig. 3 to Fig. 5. First, as shown in Fig. 3, if the plunger rod 40 is pushed and advanced in a tip direction, an inner pressure of the rear chamber 12 is increased and, following the pressure increase, the intermediate gasket 70 is also advanced. At a point of time in which the seal part 71 of the intermediate gasket 70 has moved, passed the a base end of the bypass 13, the liquid medicine L in the rear chamber 12 flows into the front chamber 11 through the connection passage 72b and the circumferential groove 72a

As shown in Fig. 4, the plunger rod 40 is advanced until the top face of the plunger gasket 80 abuts against a bottom face of the intermediate gasket 70, thereby causing the total quantity of the liquid medicine L in the rear chamber 12 to flow into the front chamber 11. The powdery medicine D is dissolved in the liquid medicine L, thereby forming the medicine liquid LD. Thereafter, as shown in Fig. 5, the injection needle 90 is connected to the nozzle 21, and the medicine liquid LD is injected into a blood vessel and the like.

### Example 2

It is desirable that a pre-filled syringe 2 has, among the above-mentioned characteristics, (1), (2), (7) and (8), and additionally (9), (10) and (11). Concretely, as shown in Fig. 9 to Fig. 13, its basic construction is approximately the same as example 1. Differences are a shape of an intermediate gasket 270 and a shape of a bypass 213. Hereinafter, there are explained the points of difference. As shown in Fig. 10, in the intermediate gasket 270, a bypass communication passage 272 comprising a first groove 272a extending from a tip side in a base end direction and a second groove 272b extending from a base end side in a tip direction are formed symmetrically with respect to a center axis. Thus, there is formed a zigzag structure in which a tip of the second groove 272b is located in a tip side relative to a base end of the first groove 272a, and the first groove 272a and the second groove are mutually separated. Further, plural annular ribs 271a are formed in a circumferential direction of a side face of the intermediate gasket 270, these annular ribs 271a contact an inner wall of a barrel 210, and annular grooves 271b formed between the annular rib 271a and the annular ribs 271a do not mutually communicate and are separated also from the first groove 272a and also from the second groove 272b. Incidentally, the bypass 13 of example 1 is formed in the axial direction of the barrel 10, whereas the bypass 213 of example 2 is formed in a circumferential direction of the barrel 210.

Here, as shown in Fig. 12, when a length of the bypass 213 in the circumferential direction is a2, and a length of the shortest portion within a length of a seal part 271 in the circumferential direction, which is separated by the first groove 272a and the second groove 272b is b2, a2 > b2. In this manner, when the intermediate gasket 270 is moved forward, the liquid medicine L in a rear chamber 212 passes over the seal part 271, and can flow into a front chamber 211 through the bypass 213. Incidentally, the bypass 213 of example 2 is formed around the barrel 210 over more than a half circumference, so that the a2 becomes considerably long with respect to the b2. This is because, in a case where a side face in which the first groove 272a and the second groove 272b are not provided and the bypass is located on the same axis, the bypass communication passage 272 and the bypass 213 do not communicate even if the intermediate gasket 270 is advanced.

Subsequently, there is explained about a method of using the pre-filled syringe 2 of example 2 by referring to Fig. 11. If a plunger rod 240 is pushed to thereby advance the intermediate gasket 270 to a position in which the bypass 213 spans the first groove 272a and the second groove 272b, the first groove 272a and the second groove 272b communicate with the bypass 213. In this position, the liquid medicine L having been accommodated in the rear chamber 212 can flow into the front chamber 211 through the bypass 213 and the bypass communication passage 272.

Thereafter, the plunger rod 240 is advanced until a top face of a plunger gasket 280 abuts against a bottom face of the intermediate gasket 270, thereby causing the total quantity of the liquid medicine L in the rear chamber 212 to flow into the front chamber 211. The powdery medicine D is dissolved in the liquid medicine L to thereby form the medicine liquid LD, and an injection needle (not shown in the drawing) is connected to the nozzle 21 and the medicine liquid LD is injected into a blood vessel and the like.

### Example 3

Subsequently, an intermediate gasket 370 of a pre-filled syringe of example 3 is shown in Fig. 14. Although not shown in the drawing, the basic construction except for the intermediate gasket 370 of example 3 is the same as example 1, and the difference being the shape of a connection passage 372b of the intermediate gasket 370. Although bypass communication passage 372 of example 3 is constituted similarly to example 1 by a circumferential groove 372a and the connection passage 372b, the connection passage 372b of example 3 consists of one thread groove.

### Example 4

Subsequently, an intermediate gasket 470 of a pre-filled syringe of example 4 is shown in Fig. 15. Although not shown in the drawing, the basic construction except for the intermediate gasket 470 of example 4 is the same as example 1, and the difference being the shape of a connection passage 472b of the intermediate gasket 470. Although bypass communication passage 472 of example 4 is constituted similarly to example 1 by a circumferential groove 472a and the connection passage 472b, the connection passage 472b of example 4 is not a spiral thread groove as shown in example 1 and example 3, but is a linear groove extending in an axial direction.

### Example 5

Subsequently, an intermediate gasket 570 of a pre-filled syringe of example 5 is shown in Fig. 16. Although not shown in the drawing, the basic construction except for the intermediate gasket 570 of example 5 is the same as example 1, and the difference being the shape of a connection passage 572b of the intermediate gasket 570. The connection passage 572b of the intermediate gasket 570 is an approximately L-shaped conduit formed inside the intermediate gasket 570. One end is connected to a circumferential groove 572a, and the other end is connected to a bottom face of the intermediate gasket 570.

### Example 6

Subsequently, an intermediate gasket 670 of a pre-filled syringe of example 6 is shown in Fig. 17. Although not shown in the drawing, the basic construction except for the intermediate gasket 670 of example 6 is the same as example 1. A bypass passage 672 of example 6 is formed by a first thread groove 672a extending from a tip in a base end direction, and a second thread groove 672b extending from a base end in a tip direction. These two thread grooves 672a and 672b do not communicate, and a seal part 671 is formed between both. Incidentally, the seal part 671 is constituted by an annular rib 671a and an annular groove 671b. Here, when an axial length of the seal part 671 is b3, and a barrel axial length of the bypass is a3 (not shown in the drawing), a3 > b3 as in Example 1.

### Example 7

Subsequently, an intermediate gasket 770 of a pre-filled syringe of example 7 is shown in Fig. 18. Although not shown in the drawing, a basic construction except for the intermediate gasket 770 of example 7 is the same as example 1. A bypass communication passage 772 of example 7 comprises a construction including a first thread groove 772a extending from a tip in a base end direction, and a second thread groove 772b extending from a base end in a tip direction. This construction is similar to example 6, but differs from example 6 in the point that both the grooves 772a and 772b are adjacent and a depression 773 is formed in a side wall in which the bypass communication passage 772 is not formed.

## Claims

1. A pre-filled syringe (1) which comprises a barrel (10) having a tip in which a nozzle (20) is provided and an open base end, an intermediate gasket (70) liquid-tightly partitioning an inside of the barrel (10) into a front chamber (11) and a rear chamber (12), a plunger gasket (80) located in a base end side of the intermediate gasket (70) and sealing the inside of the barrel (10), and a plunger rod (40) connected to a base end of the plunger gasket (80), and in which in a tip side of the barrel (10) relative to the intermediate gasket (70) there is formed a bypass (13) protruding outwardly in a radial direction,
wherein the intermediate gasket includes a seal part (71) contacting an inner wall of the barrel (10) and liquid-tightly partitioning the front chamber (11) and the rear chamber (12), and a bypass communication passage (72) providing communication between the front chamber (11) and the rear chamber (12) in cooperation with the bypass, wherein when an axial length of the bypass (13) is a1 and an axial effective length of the seal part is b1, a1 > b1, wherein the bypass communication passage (72) includes a circumferential groove (72a) formed in an approximately circumferential direction of a base end side of the seal part (71), and a connection passage (72b) connecting the circumferential groove (72a) and the rear chamber (12), wherein powdery medicine is accommodated in the front chamber (11) and liquid medicine is accommodated in the rear chamber (12), **characterized in that** the connection passage is a spiral groove formed in an outer wall of the intermediate gasket.

2. A pre-filled syringe according to claim 1, wherein the barrel (10) additionally comprises a tip gasket (60), and the front chamber (11) is formed between the tip gasket and the intermediate gasket (70).

3. A pre-filled syringe according to claim 2, wherein the barrel additionally comprises a nozzle member (20), the nozzle is formed in a tip of the nozzle member, and the nozzle member (20) includes a tip gasket accommodation part (22) capable of accommodating the tip gasket (60), and a liquid passing passage (23) through which a liquid medicine can pass when the tip gasket (60) has been accommodated in the tip gasket accommodation part (22).

4. A pre-filled syringe according to claim 3, wherein if an axial length of the tip gasket is A, an axial length of the intermediate gasket is B, an axial length of the plunger gasket is C and a length from an inner wall tip of the nozzle member to an inner wall base end of the bypass is D, A + B + C > D.

## Patentansprüche

1. Vorgefüllte Spritze (1) umfassend einen Zylinder (10) mit einer Spitze, die eine Düse (20) und ein offenes unteres Ende aufweist, eine Zwischendichtung (70) zum flüssigkeitsdichten Abteilen des Inneren des Zylinders (10) in eine vordere Kammer (11) und eine hintere Kammer (12), eine Kolbendichtung (80), die an einem unteren Ende der Zwischendichtung (70) angeordnet ist und das Innere des Zylinders (10) abdichtet, und eine Kolbenstange (40), die mit einem unteren Ende der Kolbendichtung (80) verbunden ist, und wobei an einer Spitzenseite des Zylinders (10) relativ zu der Zwischendichtung (70) eine Umgehung (13) in radialer Richtung nach außen ausgebildet ist,
wobei die Zwischendichtung einen Dichtungsteil (71) umfasst, der in Kontakt mit einer inneren Wand des Zylinders (10) steht und die vordere Kammer (11) und die hintere Kammer (12) flüssigkeitsdicht abteilt, und einen Umgehungsverbindungsdurchgang (72), der eine Verbindung zwischen der vorderen Kammer (11) und der hinteren Kammer (12) in Verbindung mit der Umgehung bereitstellt, wobei, wenn eine axiale Länge der Umgehung (13) a1 ist und eine axiale tatsächliche Länge des Dichtungsteils b1 ist, a1 > b1 ist, wobei der Umgehungsverbindungsdurchgang (72) eine umlaufende Vertiefung (72a) beinhaltet, die annähernd in Umfangsrichtung einer Seite eines unteren Endes des Dichtungsteils (71) ausgebildet ist, und einen Verbindungsdurchgang (72b), der die umlaufende Vertiefung (72a) und die hintere Kammer (12) verbindet, wobei sich ein pulverförmiges Medikament in der vorderen Kammer (11) und ein flüssiges Medikament in der hinteren Kammer (12) befindet,
**dadurch gekennzeichnet, dass** der Verbindungsdurchgang eine spiralförmige Vertiefung ist, die in einer äußeren Wand der Zwischendichtung ausgebildet ist.

2. Vorgefüllte Spritze nach Anspruch 1, wobei der Zylinder (10) weiterhin eine Spitzendichtung (60) umfasst, und die vordere Kammer (11) zwischen der Spitzendichtung und der Zwischendichtung (70) ausgebildet ist.

3. Vorgefüllte Spritze nach Anspruch 2, wobei der Zylinder weiterhin ein Düsenelement (20) umfasst, die Düse in einer Spitze des Düsenelements ausgebildet ist, und das Düsenelement (20) ein Spitzendichtungsaufnahmeteil (22) beinhaltet, das die Spitzendichtung (60) aufnehmen kann, und einen Flüssigkeitsdurchgang (23), durch den ein flüssiges Medikament passieren kann, wenn die Spitzendichtung (60) in dem Spitzendichtungsaufnahmeteil (22) aufgenommen ist.

4. Vorgefüllte Spritze nach Anspruch 3, wobei, wenn eine axiale Länge der Spitzendichtung A ist, eine axiale Länge der Zwischendichtung B ist, eine axiale Länge der Kolbendichtung C ist und eine Länge von einer Spitze einer inneren Wand des Düsenelements zum unteren Ende einer inneren Wand der Umgehung D ist, A + B + C > D ist.

## Revendications

1. Seringue pré-remplie (1) comprenant un corps (10) présentant un bout dans lequel est prévue une buse (20) et une extrémité de base ouverte, un joint intermédiaire (70) divisant un côté intérieur du corps (10) de manière imperméable aux liquides en une chambre avant (11) et une chambre arrière (12), une garniture de piston (80) localisée dans une face d'extrémité de base du joint intermédiaire (70) et étanchant l'intérieur du corps (10), et un tige de piston (40) connectée à une extrémité de base de la garniture de piston (80), et dans lequel, dans une face de bout du corps (10) par rapport au joint intermédiaire (70), est formée une dérivation (13) faisant saillie vers l'extérieur dans une direction radiale,
le joint intermédiaire incluant une partie de joint d'étanchéité (71) en contact avec une paroi intérieure du corps (10) et divisant de manière imperméable aux liquides la chambre avant (11) et la chambre arrière (12) et un passage de communication de dérivation (72) fournissant une communication entre la chambre avant (11) et la chambre arrière (12) en coopération avec la dérivation, une longueur axiale de la dérivation (13) étant a1, et une longueur axiale effective de la partie de joint d'étanchéité étant b1, a1 > b1, le passage de communication de dérivation (72) incluant une rainure circonférentielle (72a) formée dans une direction approximativement circonférentielle d'une face d'extrémité de base de la partie de joint d'étanchéité (71) et un passage de connexion (72b) connectant la rainure circonférentielle (72a) et la chambre arrière (12), une médicine sous forme de poudre étant logée dans la chambre avant (11) et une médicine liquide étant logée dans la chambre arrière(12),
**caractérisée en ce que** le passage de connexion est une rainure hélicoïdale formée dans une paroi extérieure du joint intermédiaire.

2. Seringue pré-remplie selon la revendication 1, le corps (10) comprenant en outre un joint de bout (60), et la chambre avant (11) étant formée entre le joint de bout et le joint intermédiaire (70).

3. Seringue pré-remplie selon la revendication 2, le corps comprenant en outre un élément de buse (20), la buse étant formée dans un bout de l'élément de buse, et l'élément de buse (20) incluant une partie de logement (22) du joint de bout apte à loger le joint de bout (60), et un passage perméable aux liquides (23) à travers lequel une médicine liquide peut passer lorsque le joint de bout (60) a été logé dans la partie de logement (22) du joint de bout.

4. Seringue pré-remplie selon la revendication 3, dans lequel, si une longueur axiale du joint de bout est A, une longueur axiale du joint d'étanchéité intermédiaire est B, une longueur axiale de la garniture de piston est C, et une longueur d'un bout de paroi intérieure de l'élément de buse vers une extrémité de base de la dérivation est D, A + B + C > D.
